# EUROPÄISCHE PATENTANMELDUNG

(11) **EP 0 830 841 A1**
(43) Veröffentlichungstag der Anmeldung: **25.03.1998**
(21) Anmeldenummer: 97116314.2
(22) Anmeldetag: 19.09.1997
(51) Int. Cl.: A61B 5/03

(54) **Messvorrichtung für medizinische Anwendungen mit einem intrakorporal einsetzbaren Sensorelement und Verfahren zum Herstellen einer Messvorrichtung**

(30) Priorität: 20.09.1996 DE 19638813
(71) Anmelder: SICAN F&E GmbH ( SIBET), 30419 Hannover (DE)
(72) Erfinder: Brehmeier-Flick, Bernd, 31737 Rinteln (DE); Runge, Lorenz, 30419 Hannover (DE); Krause, Jens, Dr., 30826 Garbsen (DE)

(57) **Zusammenfassung**

Drucksensoren weisen eine Metallhülse auf, in die ein Zuleitungskabel mit einem angeschlossenen Drucksensor eingebracht ist. Die einzelnen Leitungen werden um den Drucksensor zur Zugentlastung herumgeführt, auf den Drucksensor gebondet und mit einem Kunststoff vergossen.
Die Meßvorrichtung hat eine flexible Folienleiterbahn (Tape) (1) zur elektrischen und mechanischen Verbindung zwischen der Auswerteeinheit und dem Sensorelement (5). Es weist weiterhin ein Substrat (4) im Bereich des Sensorelements (5) auf, das mit dem einen Ende (3) der Folienleitbahn (1) mechanisch verbunden ist und eine höhere mechanische Festigkeit als die Folienleiterbahn (1) hat.
Die Meßvorrichtung kann z.B. zur Hirndruckmessung verwendet werden.

## Beschreibung

In medizinischen Anwendungen werden Meßsonden mit Hilfe eines Katheters in den Körper (intrakorporal) eingeführt und an Stellen geleitet, an denen Biosignale gemessen werden müssen. Die Sonden müssen bei Messungen im Schädel (intrakraniell) einen sehr kleinen Querschnitt aufweisen und sind daher bevorzugt Halbleitersensoren, die in eine Trägerhülse montiert und kontaktiert sind.

Zum Beispiel wird zur Diagnose eines Wasserkopfes (Hydrozephalus) in der Klinik auf der Intensivstation der Hirndruck mit einer Sonde intrakraniell gemessen. Anschließend wird die Sonde herausgezogen und bei Einmalsonden vernichtet bzw. bei mehrfach verwendbaren Sonden sterilisiert und beim nächsten Patienten wiederverwendet.

Wenn z.B. ein Hydrozephalus diagnostiziert wurde, wird ein sog. Shunt-System gelegt, durch das bei Ansteigen des Hirndrucks über einen festgelegten Wert Gehirnwasser (Liquor) in die Bauchhöhle abgeleitet und damit ein Überdruck im Gehirn vermieden wird.

Die Hirndruckmessung kann sowohl epi- als auch subdural erfolgen. Epidural bedeutet, daß zwischen der harten Hirnhaut (Dura mater) und der Schädeldecke (Kalotte) der Hirndruck indirekt über den vom Liquor auf die Hirnhaut ausgeübten Druck bestimmt wird.

Dieser Meßort hat die Vorteile, daß die harte Hirnhaut nicht durchstoßen wird, somit eine Infektion der Hirnhaut vermieden wird, der Eingriff wesentlich einfacher ist, kein Hirngewebe bei dieser Messung verletzt wird und der Sensor einen längeren Zeitraum an seinem Meßort bleiben kann.
Eine subdurale Messung bedeutet, daß der Sensor unter die Hirnhaut geschoben wird und diese hierbei durchstoßen werden muß. Im weiteren kann nun auch der Druck im Hirngewebe (parenchymal) gemessen werden und es wird häufig das Hirngewebe durchstoßen, um eine Messung im Ventrikel (intraventrikulär) zu ermöglichen.

Es sind verschiedene intrakranielle Meßsysteme bekannt. Zum Beispiel bietet die Firma B.Braun Melsungen AG ein epidurales Meßsystem unter dem Namen 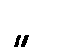 Epidyn" an. Hier ist ein Halbleiterdrucksensor in einem metallischen Gehäuse befestigt. Der Sensor ist mit Litzen eines Kabels verbunden, durch die elektrische Signale an eine extrakorporale Auswerteeinheit geleitet werden. Dieses System weist hohe hohe Fertigungskosten auf. Außerdem liegt der minimale Krümmungsradius im Zentimeterbereich. Wird dieser Radius unterschritten, so besteht die Gefahr, daß die Litzen brechen. Für medizinische Anwendungen sind jedoch geringere Krümmungsradien notwendig, da sich die Patienten im Krankenbett bewegen und gewaschen werden müssen.

Bei herkömmlichen Drucksensoren wird eine Metallhülse gefertigt, in die das Kabel samt Drucksensor geschoben wird. Die einzelnen Leitungen werden um den Drucksensor zur Zugentlastung herumgeführt, auf den Drucksensor gebondet und mit einem Kunststoff vergossen. Dieser Prozeß ist durch die geringen Abmessungen nur mit großem Aufwand an feinwerktechnischen Geräten durchführbar.

In der US 4,738,267 ist ein implantierbarer, intrakranieller Drucksensor dargestellt, wobei die elektrischen Leitungen zum Verbinden des Sensorelementes mit einer extrakorporalen Auswerteeinheit als ummantelte Litzen ausgebildet sind. Diese müssen bei der Herstellung aufwendig verbunden werden. Zudem besteht die Gefahr des Reißens der Kabelfasern bei Zugbelastung und durch die notwendigen kleinen Biegeradien die Gefahr, daß die Litzen brechen.

In der DE 43 15 987 C1 ist eine extrakorporal verwendete Körperelektrode gezeigt, bei der ein Sensor einstückig auf eine flexible Trägerfolie aufgebracht ist. Zum Anschluß einer Auswerteeinheit wird an das Ende der Trägerfolie mit einer Klemmvorrichtung ein Kabel angeschlossen. Die extrakorporale Körperelektrode ist ausschließlich zur Spannungsmessung geeignet und darf nicht intrakorporal appliziert werden. Sie wäre auch nicht geeignet, intrakorporale chemische, biologische und physikalische Sensorik zu übernehmen.

In der US 5,263,244 ist ein Verfahren zur Herstellung einer flexiblen Halbleitersensoranordnung zur optischen Pulsmessung beschrieben. Auf einer flexiblen Folie, die elektrisch leitfähige Bahnen aufweist, werden integrierte Sensorelemente in herkömmlicher Befestigungstechnik aufgebracht. Die Sensorelemente, z.B. Leuchtdioden und Photodetektoren, werden mit einer SMD-Technik mit den leitfähigen Bahnen verbunden. Die Enden der relativ kurzen Bahnen bilden Anschlüsse, an die umhüllte Litzen gelötet werden. Hierzu ist ein relativ aufwendiger Lötprozeß erforderlich, wobei die Lötstellen ein Qualitätsrisiko darstellen. Zudem besteht die Gefahr, daß die Litzen bei engen Biegeradien brechen. Außerdem ist es nicht möglich, integrierte Drucksensoren mit SMD-Technik auf die entsprechenden Anschlüsse der leitfähigen Bahnen zu löten.

### Aufgabe

Aufgabe der Erfindung war es, eine Meßvorrichtung für medizinische Anwendungen mit einem intrakorporal einsetzbaren Sensorelement, einer extrakorporalen Auswerteeinheit und elektrischen Verbindungsleitungen zwischen dem Sensorelement und der Auswerteeinheit zu schaffen, die zuverlässig ist, leicht und kostengünstig zu fertigen ist und dem Patienten Bewegungen im Bett erlaubt.

Die Aufgabe wird durch das Meßsystem mit den Merkmalen des Anspruchs 1 gelöst. Die Fertigung des Meßsystems kann durch das automatisierbare Verfahren nach Anspruch 5 einfach und kostengünstig erfolgen.

Das erfindungsgemäße Meßsystem ist wesentlich günstiger zu produzieren und weist eine größere Zugfestigkeit bei geringerem zulässigen Krümmungsradius auf. Bewegung und Scherung des Sensors auf der Leitbahn werden durch die Verstärkung des Folienendes mit einem plattenförmigen Substrat kompensiert.

### Zeichnungen

Die Erfindung wird mit der beigefügten Zeichnung erläutert.

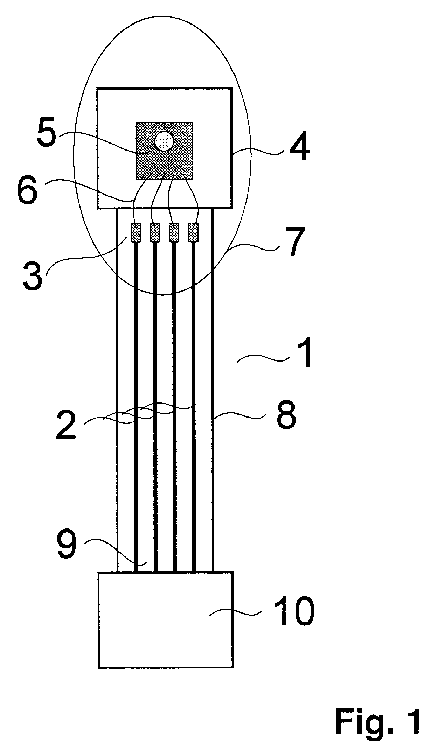

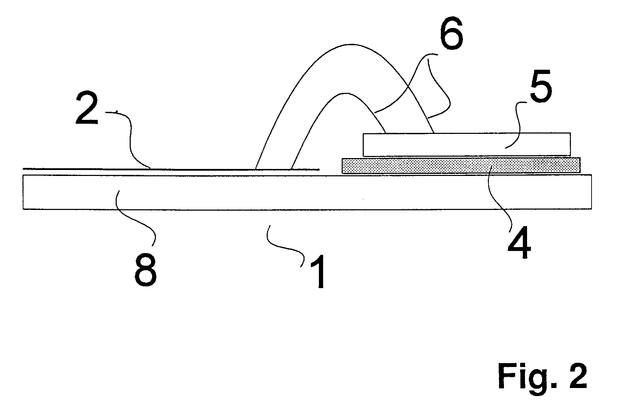

- Fig. 1:: Intrakorporal einsetzbares Meßsystem.

### Ausführungsbeispiel

Als bevorzugtes Ausführungsbeispiel wird ein Hirndruckmeßsystem vorgestellt. Gleichermaßen kann die Aufbautechnik aber auch für andere medizinische Anwendungen verwendet werden.

In der Figur 1 ist ein intrakorporales Hirndruckmeßsystem dargestellt. Als Leitungsersatz für die Drähte zur Energiezuführung und zur Meßsignalübertragung wird eine flexible Leiterbahn (Tape) 1 mit elektrischen Leitern 2 verwendet. An dem einen Ende des Kabels 3 befindet sich ein Substrat 4, das die Aufbautechnik des Sensorelementes verstärkt. Der Drucksensor 5 wird auf das Substrat 4 aufgeklebt und mit Verbindungsdrähten 6 gebondet. Danach werden die Bonddrähte des Drucksensors 5 mit einer Vergußmasse 7 fixiert. Anschließend wird der Drucksensor 5 mit dem Kabelende 3 in eine Masse 7 aus ein Polyimid oder Polyurethan eingegossen bzw. mit einem Silikonmantel versehen. Bevorzugt wird hierzu eine Silikonmasse verwendet, da dies ein flexibles Material ist, durch das der Druck übertragen werden kann. Außerdem bietet es eine sichere Isolation gegen elektrische Ströme. Dies ist besonders bei medizinischen Anwendungen sehr wichtig, um den Patienten zu schützen. Das Trägermaterial 8 der flexiblen Leiterbahn 1 ist z.B. eine FR4-Folie. Das Substrat 4 zur Verstärkung des Kabelendes 3 besteht derzeit ebenfalls aus einem FR4-Substrat. An dem zweiten Kontaktende 9 der flexiblen Leiterbahn 1 befindet sich ein Steckkontakt 10 oder in weiterer Entwicklung eine elektronische Schaltung auf die die Abgleichschaltung aufgebracht wird.

Zur Herstellung der flexiblen Leiterbahn (Tapekabel) 1 wird die Trägerfolie 7 mit herkömmlichen Verfahren belichtet, geätzt und gestanzt.

An das zweite Kontaktende 9 wird ein Steckkontakt 10 angebracht. Alternativ dazu könnten die Leitbahnen 2 auch aufgefächert werden und die Platine mit der Auswertschaltung direkt auf die Leiter 2 gefertigt werden.

Zur intraventrikulären Messung des Hirndrucks wird die Hirnhaut durchstochen und der Sensor 5 mit dem Tapekabel durch die Gehirnmasse in den Ventrikel geführt. Der Ventrikel ist der Ort im Gehirn, der im Liquorkreislauf des Gehirns kurz vor dem Rückenmark liegt. Im Falle eines Hydrozephalus, ist der Ventrikel erweitert und dessen Abfluß verstopft und macht somit die Rückenmarkskanalresorption des Liquors unmöglich, so daß der dort herrschende Druck ein sehr genaues und zuverlässiges Indiz für eine Störung des Liquorkreislaufes ist. Der Hauptvorteil einer intraventrikulären Hirndruckmessung besteht in der höheren Meßgenauigkeit.

## Patentansprüche

1. Meßvorrichtung für medizinische Anwendungen mit einem intrakorporal einsetzbaren Sensorelement (5), einer extrakorporalen Auswerteeinheit und elektrischen Verbindungsleitungen (2, 6) zwischen dem Sensorelement (5) und der Auswerteeinheit,
**dadurch gekennzeichnet**, daß
- eine flexible Folienleiterbahn (1) zum elektrischen und mechanischen Verbinden der Auswerteeinheit mit dem Sensorelement (5) vorgesehen ist,
- ein Substrat (4) im Bereich des Sensorelements (5) mit dem einen Ende (3) der Folienleiterbahn (1) mechanisch verbunden ist,
- das Substrat (4) eine höhere mechanische Festigkeit aufweist als die Folienleiterbahn (1),
- das Sensorelement (5) mit elektrischen Leitern (2) der flexiblen Folienleiterbahn (1) verbunden ist,
- das Substrat (4) mit dem Sensorelement (5) in eine flexible Masse (7) eingeschlossen ist.

2. Meßvorrichtung nach Anspruch 1, **dadurch gekennzeichnet**, daß das Trägermaterial (8) der Folienleiterbahn (1) aus dem Material FR4 besteht.

3. Meßvorrichtung nach einem der vorhergehenden Ansprüche, **dadurch gekennzeichnet**, daß das Substrat (4) ein FR4-Substrat ist.

4. Meßvorrichtung nach einem der vorhergehenden Ansprüche, **dadurch gekennzeichnet**, daß das Sensorelement (5) ein Drucksensor oder ein kombinierter Druck- und Temperatursensor ist.

5. Verfahren zum Herstellen einer Meßvorrichtung gemäß einem der Ansprüche 1 bis 4, umfassend die Schritte:
- Herstellen einer flexiblen Folienleiterbahn (1);
- Mechanisches Verbinden eines Substrats (4) mit dem einen Ende (3) der Folienleiterbahn (1);
- Befestigen des Sensorelements (5) auf dem Substrat (4) oder an dem einen Ende (3) der Folienleiterbahn (1);
- Elektrisches Verbinden des Sensorelements (5) mit Leitern (2) der Folienleiterbahn (1);
- Implementierung der Auswerteeinheit an dem anderen Ende der Folienleiterbahn (1);
- Einbinden des Substrats (4) mit dem Sensorelement (5) in eine flexible Masse (7).
